(19) Europäisches Patentamt
European Patent Office
Office européen des brevets

(11) EP 1 390 531 B1

(12) **EUROPÄISCHE PATENTSCHRIFT**

(45) Veröffentlichungstag und Bekanntmachung des Hinweises auf die Patenterteilung:
**21.06.2006 Patentblatt 2006/25**

(21) Anmeldenummer: **02712954.3**

(22) Anmeldetag: **21.03.2002**

(51) Int Cl.:
***C12Q 1/68*** *(2006.01)* ***G01N 33/573*** *(2006.01)*
***G01N 33/68*** *(2006.01)*

(86) Internationale Anmeldenummer:
**PCT/EP2002/003180**

(87) Internationale Veröffentlichungsnummer:
**WO 2002/074987 (26.09.2002 Gazette 2002/39)**

(54) **QUANTITATIVE DIAGNOSTISCHE ANALYSE DER PRÄDISPOSITION FÜR HYPERTONIE**
QUANTITATIVE DIAGNOSTIC ANALYSIS OF PREDISPOSITION TO HYPERTENSION
ANALYSE DIAGNOSTIQUE QUANTITATIVE DE LA PRÉDISPOSITION À L'HYPERTENSION

(84) Benannte Vertragsstaaten:
**AT BE CH CY DE DK ES FI FR GB GR IE IT LI LU MC NL PT SE TR**

(30) Priorität: **21.03.2001 DE 10113876**

(43) Veröffentlichungstag der Anmeldung:
**25.02.2004 Patentblatt 2004/09**

(73) Patentinhaber: **Lang, Florian**
**72076 Tübingen (DE)**

(72) Erfinder:
- **LANG, Florian**
  **72076 Tübingen (DE)**
- **BUSJAHN, Andreas,**
  **Charité Universitätsklinikum**
  **10117 Berlin (DE)**
- **LUFT, Friedrich, C.**
  **13125 Berlin (DE)**

(74) Vertreter: **Isenbruck, Günter**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
**WO-A-00/62781**

- **WAERNTGES S ET AL: "Transcriptional upregulation of the human serine/threonine kinase hSGK1 in glomerulonephritis." KIDNEY & BLOOD PRESSURE RESEARCH, Bd. 23, Nr. 3-5, 2000, Seite 246 XP008016819 Congress of Nephrology 2000;Vienna, Austria; September 02-05, 2000 ISSN: 1420-4096**
- **DATABASE ON-LINE MEDICAL DICTIONAR [Online] 12. Dezember 1998 (1998-12-12) XP002240465**
- **FATTORUSO, V ET AL: "Vademecum Clinique" 2001 , MASSON , PARIS XP002240464 Seite 1493, rechte Spalte, Zeile 31 -Seite 1494, linke Spalte, Zeile 25**
- **NEPHROL. DIAL. TRANSPLANT. Bd. 19, 2004, Seiten 2499 - 2504**
- **TODOROVA A. ET AL J.MED.GENET. Bd. 40, Nr. E115, 2003, Seiten 1 - 3**
- **RICHARDS ET AL NATURE GENETICS Bd. 10, Juli 1995, Seite 259**
- **DATABASE [Online] 'Auch stille Mutationen können zu Krankheiten führen' Retrieved from HTTP://HOME.T-ONLINE.DE/HOME/ LINDE.PETERS/G ENGEF04.HTM**

Bemerkungen:
Die Akte enthält technische Angaben, die nach dem Eingang der Anmeldung eingereicht wurden und die nicht in dieser Patentschrift enthalten sind.

EP 1 390 531 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

SEQUENCE LISTING

<110> Lang, Florian

<120> Quantitative diagnostische Analyse der Hypertonie

<130> L61882

<140> DE 101 13 876.8
<141> 2001-03-21

<160> 2

<170> PatentIn version 3.1

<210> 1
<211> 2354
<212> DNA
<213> homo sapiens

<220>
<221> CDS
<222> (43)..(1335)
<223>

<220>
<221> variation
<222> (762)..(762)
<223> 1. SNP (C in T ), stumme Mutation, d.h. beide Versionen des SNPs resultieren in der Aminosäure Asp in der Aminosäure- Position 240

<400> 1

```
ggtctttgag cgctaacgtc tttctgtctc cccgcggtgg tg atg acg gtg aaa        54
                                               Met Thr Val Lys
                                               1

act gag gct gct aag ggc acc ctc act tac tcc agg atg agg ggc atg      102
Thr Glu Ala Ala Lys Gly Thr Leu Thr Tyr Ser Arg Met Arg Gly Met
5                   10                  15                  20

gtg gca att ctc atc gct ttc atg aag cag agg agg atg ggt ctg aac      150
Val Ala Ile Leu Ile Ala Phe Met Lys Gln Arg Arg Met Gly Leu Asn
                25                  30                  35

gac ttt att cag aag att gcc aat aac tcc tat gca tgc aaa cac cct      198
Asp Phe Ile Gln Lys Ile Ala Asn Asn Ser Tyr Ala Cys Lys His Pro
            40                  45                  50

gaa gtt cag tcc atc ttg aag atc tcc caa cct cag gag cct gag ctt      246
Glu Val Gln Ser Ile Leu Lys Ile Ser Gln Pro Gln Glu Pro Glu Leu
        55                  60                  65

atg aat gcc aac cct tct cct cca cca agt cct tct cag caa atc aac      294
Met Asn Ala Asn Pro Ser Pro Pro Pro Ser Pro Ser Gln Gln Ile Asn
    70                  75                  80
```

```
ctt ggc ccg tcg tcc aat cct cat gct aaa cca tct gac ttt cac ttc      342
Leu Gly Pro Ser Ser Asn Pro His Ala Lys Pro Ser Asp Phe His Phe
85                  90                  95                  100

ttg aaa gtg atc gga aag ggc agt ttt gga aag gtt ctt cta gca aga      390
Leu Lys Val Ile Gly Lys Gly Ser Phe Gly Lys Val Leu Leu Ala Arg
                105                 110                 115

cac aag gca gaa gaa gtg ttc tat gca gtc aaa gtt tta cag aag aaa      438
His Lys Ala Glu Glu Val Phe Tyr Ala Val Lys Val Leu Gln Lys Lys
            120                 125                 130

gca atc ctg aaa aag aaa gag gag aag cat att atg tcg gag cgg aat      486
Ala Ile Leu Lys Lys Lys Glu Glu Lys His Ile Met Ser Glu Arg Asn
        135                 140                 145

gtt ctg ttg aag aat gtg aag cac cct ttc ctg gtg ggc ctt cac ttc      534
Val Leu Leu Lys Asn Val Lys His Pro Phe Leu Val Gly Leu His Phe
    150                 155                 160

tct ttc cag act gct gac aaa ttg tac ttt gtc cta gac tac att aat      582
Ser Phe Gln Thr Ala Asp Lys Leu Tyr Phe Val Leu Asp Tyr Ile Asn
165                 170                 175                 180

ggt gga gag ttg ttc tac cat ctc cag agg gaa cgc tgc ttc ctg gaa      630
Gly Gly Glu Leu Phe Tyr His Leu Gln Arg Glu Arg Cys Phe Leu Glu
                185                 190                 195

cca cgg gct cgt ttc tat gct gct gaa ata gcc agt gcc ttg ggc tac      678
Pro Arg Ala Arg Phe Tyr Ala Ala Glu Ile Ala Ser Ala Leu Gly Tyr
            200                 205                 210

ctg cat tca ctg aac atc gtt tat aga gac tta aaa cca gag aat att      726
Leu His Ser Leu Asn Ile Val Tyr Arg Asp Leu Lys Pro Glu Asn Ile
        215                 220                 225

ttg cta gat tca cag gga cac att gtc ctt act gac ttc gga ctc tgc      774
Leu Leu Asp Ser Gln Gly His Ile Val Leu Thr Asp Phe Gly Leu Cys
    230                 235                 240

aag gag aac att gaa cac aac agc aca aca tcc acc ttc tgt ggc acg      822
Lys Glu Asn Ile Glu His Asn Ser Thr Thr Ser Thr Phe Cys Gly Thr
245                 250                 255                 260

ccg gag tat ctc gca cct gag gtg ctt cat aag cag cct tat gac agg      870
Pro Glu Tyr Leu Ala Pro Glu Val Leu His Lys Gln Pro Tyr Asp Arg
                265                 270                 275

act gtg gac tgg tgg tgc ctg gga gct gtc ttg tat gag atg ctg tat      918
Thr Val Asp Trp Trp Cys Leu Gly Ala Val Leu Tyr Glu Met Leu Tyr
            280                 285                 290

ggc ctg ccg cct ttt tat agc cga aac aca gct gaa atg tac gac aac      966
Gly Leu Pro Pro Phe Tyr Ser Arg Asn Thr Ala Glu Met Tyr Asp Asn
        295                 300                 305
```

```
att ctg aac aag cct ctc cag ctg aaa cca aat att aca aat tcc gca      1014
Ile Leu Asn Lys Pro Leu Gln Leu Lys Pro Asn Ile Thr Asn Ser Ala
    310                 315                     320

aga cac ctc ctg gag ggc ctc ctg cag aag gac agg aca aag cgg ctc      1062
Arg His Leu Leu Glu Gly Leu Leu Gln Lys Asp Arg Thr Lys Arg Leu
325                 330                     335                 340

ggg gcc aag gat gac ttc atg gag att aag agt cat gtc ttc ttc tcc      1110
Gly Ala Lys Asp Asp Phe Met Glu Ile Lys Ser His Val Phe Phe Ser
                345                     350                 355

tta att aac tgg gat gat ctc att aat aag aag att act ccc cct ttt      1158
Leu Ile Asn Trp Asp Asp Leu Ile Asn Lys Lys Ile Thr Pro Pro Phe
            360                     365                 370

aac cca aat gtg agt ggg ccc aac gac cta cgg cac ttt gac ccc gag      1206
Asn Pro Asn Val Ser Gly Pro Asn Asp Leu Arg His Phe Asp Pro Glu
        375                     380                 385

ttt acc gaa gag cct gtc ccc aac tcc att ggc aag tcc cct gac agc      1254
Phe Thr Glu Glu Pro Val Pro Asn Ser Ile Gly Lys Ser Pro Asp Ser
    390                 395                     400

gtc ctc gtc aca gcc agc gtc aag gaa gct gcc gag gct ttc cta ggc      1302
Val Leu Val Thr Ala Ser Val Lys Glu Ala Ala Glu Ala Phe Leu Gly
405                 410                     415                 420

ttt tcc tat gcg cct ccc acg gac tct ttc ctc tgaaccctgt tagggcttgg    1355
Phe Ser Tyr Ala Pro Pro Thr Asp Ser Phe Leu
                425                     430

ttttaaagga ttttatgtgt gtttccgaat gttttagtta gccttttggt ggagccgcca    1415

gctgacagga catcttacaa gagaatttgc acatctctgg aagcttagca atcttattgc    1475

acactgttcg ctggaagctt tttgaagagc acattctcct cagtgagctc atgaggtttt    1535

catttttatt cttccttcca acgtggtgct atctctgaaa cgagcgttag agtgccgcct    1595

tagacggagg caggagtttc gttagaaagc ggacgctgtt ctaaaaaagg tctcctgcag    1655

atctgtctgg gctgtgatga cgaatattat gaaatgtgcc ttttctgaag agattgtgtt    1715

agctccaaag cttttcctat cgcagtgttt cagttcttta ttttcccttg tggatatgct    1775

gtgtgaaccg tcgtgtgagt gtggtatgcc tgatcacaga tggattttgt tataagcatc    1835

aatgtgacac ttgcaggaca ctacaacgtg ggacattgtt tgtttcttcc atatttggaa    1895

gataaattta tgtgtagact tttttgtaag atacggttaa taactaaaat ttattgaaat    1955

ggtcttgcaa tgactcgtat tcagatgctt aaagaaagca ttgctgctac aaatatttct    2015

atttttagaa agggttttta tggaccaatg ccccagttgt cagtcagagc cgttggtgtt    2075

tttcattgtt taaaatgtca cctgtaaaat gggcattatt tatgtttttt tttttgcatt    2135
```

```
cctgataatt gtatgtattg tataaagaac gtctgtacat tgggttataa cactagtata    2195
tttaaactta caggcttatt tgtaatgtaa accaccattt taatgtactg taattaacat    2255
ggttataata cgtacaatcc ttccctcatc ccatcacaca actttttttg tgtgtgataa    2315
actgattttg gtttgcaata aaaccttgaa aaatattta                           2354
```

<210>' 2
<211> 431
<212> PRT
<213> homo sapiens

<400> 2

```
Met Thr Val Lys Thr Glu Ala Ala Lys Gly Thr Leu Thr Tyr Ser Arg
1               5                   10                  15

Met Arg Gly Met Val Ala Ile Leu Ile Ala Phe Met Lys Gln Arg Arg
            20                  25                  30

Met Gly Leu Asn Asp Phe Ile Gln Lys Ile Ala Asn Asn Ser Tyr Ala
        35                  40                  45

Cys Lys His Pro Glu Val Gln Ser Ile Leu Lys Ile Ser Gln Pro Gln
    50                  55                  60

Glu Pro Glu Leu Met Asn Ala Asn Pro Ser Pro Pro Pro Ser Pro Ser
65                  70                  75                  80

Gln Gln Ile Asn Leu Gly Pro Ser Ser Asn Pro His Ala Lys Pro Ser
                85                  90                  95

Asp Phe His Phe Leu Lys Val Ile Gly Lys Gly Ser Phe Gly Lys Val
            100                 105                 110

Leu Leu Ala Arg His Lys Ala Glu Glu Val Phe Tyr Ala Val Lys Val
        115                 120                 125

Leu Gln Lys Lys Ala Ile Leu Lys Lys Lys Glu Glu Lys His Ile Met
    130                 135                 140

Ser Glu Arg Asn Val Leu Leu Lys Asn Val Lys His Pro Phe Leu Val
145                 150                 155                 160
```

```
Gly Leu His Phe Ser Phe Gln Thr Ala Asp Lys Leu Tyr Phe Val Leu
                165                 170                 175

Asp Tyr Ile Asn Gly Gly Glu Leu Phe Tyr His Leu Gln Arg Glu Arg
            180                 185                 190

Cys Phe Leu Glu Pro Arg Ala Arg Phe Tyr Ala Ala Glu Ile Ala Ser
        195                 200                 205

Ala Leu Gly Tyr Leu His Ser Leu Asn Ile Val Tyr Arg Asp Leu Lys
    210                 215                 220

Pro Glu Asn Ile Leu Leu Asp Ser Gln Gly His Ile Val Leu Thr Asp
225                 230                 235                 240

Phe Gly Leu Cys Lys Glu Asn Ile Glu His Asn Ser Thr Thr Ser Thr
                245                 250                 255

Phe Cys Gly Thr Pro Glu Tyr Leu Ala Pro Glu Val Leu His Lys Gln
            260                 265                 270

Pro Tyr Asp Arg Thr Val Asp Trp Trp Cys Leu Gly Ala Val Leu Tyr
        275                 280                 285

Glu Met Leu Tyr Gly Leu Pro Pro Phe Tyr Ser Arg Asn Thr Ala Glu
    290                 295                 300

Met Tyr Asp Asn Ile Leu Asn Lys Pro Leu Gln Leu Lys Pro Asn Ile
305                 310                 315                 320

Thr Asn Ser Ala Arg His Leu Leu Glu Gly Leu Leu Gln Lys Asp Arg
                325                 330                 335

Thr Lys Arg Leu Gly Ala Lys Asp Asp Phe Met Glu Ile Lys Ser His
            340                 345                 350

Val Phe Phe Ser Leu Ile Asn Trp Asp Asp Leu Ile Asn Lys Lys Ile
        355                 360                 365

Thr Pro Pro Phe Asn Pro Asn Val Ser Gly Pro Asn Asp Leu Arg His
    370                 375                 380

Phe Asp Pro Glu Phe Thr Glu Glu Pro Val Pro Asn Ser Ile Gly Lys
```

385 390 395 400

Ser Pro Asp Ser Val Leu Val Thr Ala Ser Val Lys Glu Ala Ala Glu
405 410 415

Ala Phe Leu Gly Phe Ser Tyr Ala Pro Pro Thr Asp Ser Phe Leu
420 425 430

## Beschreibung

Die vorliegende Erfindung betrifft den Nachweis eines unmittelbaren Zusammenhangs zwischen zwei unterschiedlichen Polymorphismen einzelner Nukleotide (single nucleotide polymorphisms = SNP) im hsgkl-Gen-und der genetisch bedingten Prädisposition zur Hypertonie.

Zahlreiche extrazelluläre Signale führen zu intrazellulären Phosphorylierungs/Dephosphorylierungskaskaden, um eine schnelle Übertragung dieser Signale von der Plasmamembran und ihren Rezeptoren in das Zytoplasma und den Zellkern zu gewährleisten. Die Spezifität dieser reversiblen Signaltransduktionskaskaden wird durch eine Vielzahl von einzelnen Proteinen, insbesondere von Kinasen, die eine Phosphatgruppe auf individuelle Substrate übertragen, ermöglicht.

Die Serum- und Glucocorticoid-abhängige Kinase (sgk), eine Serin/Threonin-Kinase, deren Expression durch Serum und Glucocorticoide gesteigert wird, wurde zunächst aus Rattenmammakarzinomazellen kloniert (Webster et al., 1993). Die humane Version der sgk, die hsgk1, wurde aus Leberzellen kloniert (Waldegger et al., 1997). Es zeigte sich, daß die Expression der hsgk1 durch die Regulation des Zellvolumens beeinflusst wird. Für die Expression der Ratten-sgk konnte eine solche Abhängigkeit vom Zellvolumen bisher nicht nachgewiesen werden. Weiterhin ergab sich, daß die Ratten-Kinase den epithelialen $Na^{+}$-Kanal (ENaC) stimuliert (Chen et al., 1999; Naray-Pejes-Toth et al., 1999). Der ENaC spielt wiederum eine entscheidende Rolle bei der renalen $Na^{+}$-Ausscheidung. Eine gesteigerte Aktivität des ENaC führt zu einer verstärkten renalen Retention von NatriumIonen, und auf diese Weise zur Entwicklung von Hypertonie.

Schließlich wurden zwei weitere Mitglieder der humanen sgk-Genfamilie kloniert, die hsgk2 und hsgk3 (Kobayashi et al., 1999), die beide - wie auch die hsgk1 - durch Insulin und IGF1 über den PI3 Kinase-Weg aktiviert werden. Elektrophysiologische Experimente zeigten, daß eine Coexpression der hsgk2 und hsgk3 ebenfalls eine signifikante Zunahme der Aktivität des ENaC zur Folge hat.

Aus der DE 197 08 173 A1 geht hervor, daß die hsgk1 bei vielen Krankheiten, bei denen Zellvolumenänderungen eine entscheidende pathophysiologische Rolle spielen, wie beispielsweise Hypematriämie, Hyponatriämie, Diabetes mellitus, Niereninsuffizienz, Hyperkatabolismus, hepatische Encephalopathie und mikrobielle oder virale Infektionen, ein beträchtliches diagnostisches Potential besitzt.

In der WO 00/62781 wurde bereits beschrieben, daß die hsgk1 den endothelialen $Na^{+}$-Kanal aktiviert, wodurch die renale $Na^{+}$-Resorption erhöht wird. Da diese gesteigerte renale $Na^{+}$-Resorption mit Hypertonie einhergeht, wurde hier vermutet, daß eine gesteigerte Expression der hsgk1 zur Hypertonie, eine verminderte Expression der hsgk1 letztlich zur Hypotonie führen sollte.

Auch in der nicht-vorveröffentlichten, prioritätsälteren deutschen Anmeldung mit dem Titel "sgk2 und sgk3 als diagnostische und therapeutische Targets" (interne Bezeichnung A 35 048) vom 28.08.00 wurde ein ähnlicher Zusammenhang zwischen der Überexpression bzw. Überaktivität der humanen Homologen hsgk2 und hsgk3 mit der Überaktivierung des ENaCs, der daraus resultierenden verstärkten renalen $Na^{+}$-Resorption und der sich daraus entwickelnden Hypertonie beschrieben. Weiterhin wurde bereits das diagnostische Potential der Kinasen hsgk2 und hsgk3 bezüglich der arteriellen Hypertonie diskutiert.

Unter einem humanen Homologen der sgk-Familie, welches im obigen Sinne eine funktionale molekulare Modifikation umfaßt, versteht man in diesem Zusammenhang ein Homologes der sgk-Familie, das auf eine solche Art und Weise mutiert ist, daß die Eigenschaften, insbesondere die katalytischen Eigenschaften oder auch die Substratsspezifität des entsprechenden Proteins verändert werden.

Die gestellte Aufgabe wird insbesondere dadurch gelöst, daß im Rahmen der vorliegenden Erfindung zwei unterschiedliche SNPs im hsgk1-Gen identifiziert wurden, die - wenn sie in einer bestimmten Version im hsgk1-Gen vorliegen -, beim Patienten eine eindeutige Neigung zur Hypertonie hervorrufen. Die Existenz solcher SNPs im hsgk1-Gen oder auch in den übrigen humanen Homologen der sgk-Genfamilie kann somit als diagnostischer Hinweis auf eine genetisch bedingte Prädisposition zur Ausbildung einer Hypertonie in Körperproben des Patienten nachgewiesen werden.

Beispielweise werden in einem diagnostischen Kit insbesondere solche Antikörper bereitgestellt, die spezifisch gegen solche Regionen des hsgk1-Proteins gerichtet sind, welche ein entsprechend eines spezifischen SNP im hsgk1-Gen mutiertes hsgk1-Protein-Fragment umfassen. Der Kit kann jedoch auch Antikörper gegen die häufigeren Allele des hsgk1-Gens oder der übrigen humanen Homologen der sgk-Familie enthalten, mit denen ein modifiziertes Expressionsniveau dieser Homologen bzw. der hsgk1 quantitativ nachgewiesen werden kann.

Weiterhin sind in einem solchen diagnostischen Kit vorzugsweise solche Polynukleotide enthalten, die spezifisch Regionen umfassend die eine oder andere Version eines Hypertonie-relevanten SNPs im hsgk1-Gen beinhalten und so zum Nachweis spezifischer SNPs im hsgk1-Gen des Patienten durch Hybridisierung unter stringenten Bedingungen mit genomischer DNA, cDNA oder mRNA aus Körperproben geeignet sind.

Bei einzelnen Patienten können individuelle Mutationen in den Genen hsgk1, hsgk2 oder hsgk3 auftreten, die die Expressionshöhe oder die funktionellen Eigenschaften der Kinasen hsgk1, hsgk2 oder hsgk3 modifizieren, und so zu einer genetisch verursachten Neigung zur Hypertonie führen. Solche Mutationen könnten beispielsweise in den regulatorischen Genregionen oder auch in Intron-Sequenzen des sgk-Genlocus auftreten und damit eine Überexpression der entsprechenden Kinase und eine Überaktivierung des ENaC verursachen. Andererseits könnten individuelle Unterschiede in der genetischen Ausstattung des sgk-Locus auch den kodierenden Genbereich betreffen. Mutationen im kodierenden Bereich könnten dann gegebenenfalls zu einer funktionalen Veränderung der entsprechenden Kinase, so z.B. zu modifizierten katalytischen Eigenschaften der Kinase führen. Demnach könnten beide oben beschriebenen Mutationsarten eine verstärkte Aktivierung des ENaCs und damit letztlich die Ausbildung einer genetisch bedingten Form von Hypertonie beim Patienten bewirken.

Solche Mutationen in den humanen Homologen der sgk-Familie, die die Ausbildung einer genetisch bedingten Form von Hypertonie beim Patienten bewirken, sind in der Regel sogenannte "single nucleotide polymorphisms" (SNP) entweder im Exon- oder im Intron-Bereich dieser Homologen. SNPs im Exon-Bereich der hsgk-Gene können in ihrer weniger häufig auftretenden Version - im folgenden die mutierte Version genannt - gegebenenfalls zu Aminosäureaustauschen im entsprechenden hsgk-Protein und somit zur einer funktionalen Modifikation der Kinase führen. SNPs im Intron-Bereich oder in regulatorischen Sequenzen der hsgk-Gene können in ihrer mutierten Version gegebenenfalls zu einer veränderten Expressionshöhe der entsprechenden Kinase führen.

Im Rahmen der vorliegenden Erfindung wurde eine Korrelationsstudie durchgeführt, in der der Genotyp des hsgk1-Gens verschiedener Patienten (Zwillinge) mit den an ihnen gemessenen systolischen und diastolischen Blutdruckwerten, die jeweils in verschiedenen Körperpositionen gemessen wurden (sitzend, stehend, liegend), verglichen und statistisch ausgewertet wurde.

So konnte im Rahmen der vorliegenden Erfindung gezeigt werden, daß die Anwesenheit eines (C$\rightarrow$T)-Austausches in Exon 8 (1. SNP, siehe SEQ ID NO. 1) auf beiden Allelen (homozygote TT-Träger des SNPs in Exon 8), der nicht zu einem Aminosäureaustausch auf Protein-Ebene führt (siehe SEQ ID NO. 2), zu signifikant höheren Blutdruckwerten und somit zu einer genetisch bedingten Neigung zur Hypertonie führt (Tab. 3).

Weiterhin konnte gezeigt werden, daß die Gegenwart eines (T$\rightarrow$C)-Austausches (2. SNP), welcher 551 bp entfernt vom 1. SNP in der Donor-Spleiß-Seite im Übergang von Intron 6 zu Exon 7 lokalisiert ist, in seiner homozygoten Ausführung zu geringeren Blutdruckwerten und somit zu einer geringeren genetisch bedingten Neigung zur Hypertonie führt (Tab. 3).

Da beide erfindungsgemäßen SNPs im hsgk1-Gen nicht zu Aminosäureaustauschen auf Protein-Ebene führen, wird die durch sie bedingte, stärker oder weniger stark ausgeprägte, genetische Prädisposition zur Hypertonie wahrscheinlich auf einer modifizierten Expressionshöhe des hsgkl-Gens basieren.

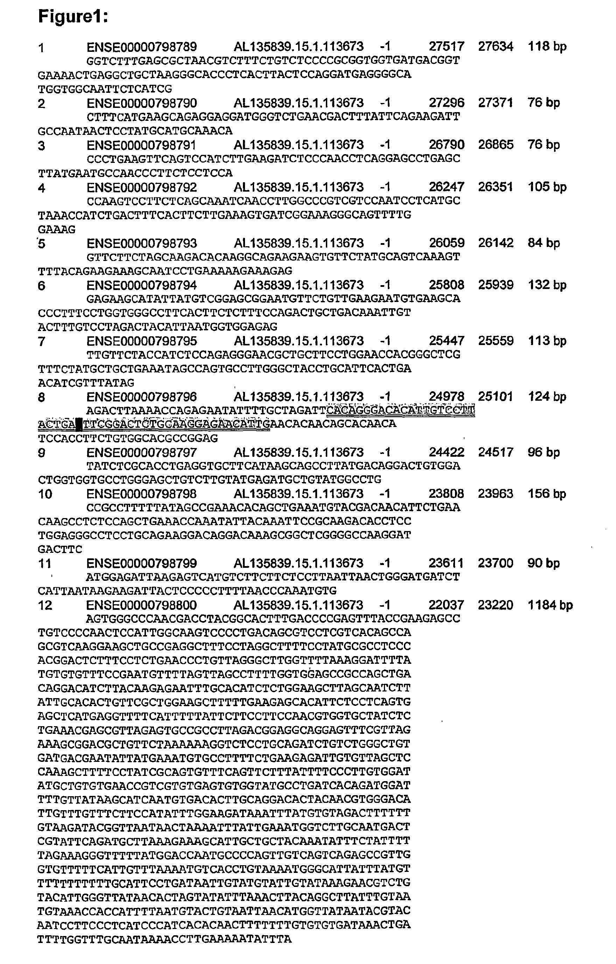

Der erste SNP in Exon 8 (C$\rightarrow$T) wird weiterhin durch Figur 1 näher erläutert. In Figur 1 werden die einzelnen Exons des hsgkl-Gens dargestellt und jeweils durch die Exon.

Nummer, den Exon-ID, den dazugehörigen "Sequenz-Contig" und Strang, sowie Start, Ende und Länge der Exons beschrieben. Die exakte Position des (C$\rightarrow$T)-Austausches im Rahmen des SNPs in Exon 8 wird durch das dunkel markierte C in Exon 8 angezeigt. Die hellere Markierung in Exon 8 in Figur 1 zeigt die SNP-flankierende Sequenz im hsgk1-Gen an, die die Position im Genom eindeutig bestimmt.

Der zweite SNP (T$\rightarrow$C) in Intron 6 wurde durch direkte Sequenzierung identifiziert, und ist dadurch eindeutig charakterisiert, daß er im hsgk1-Gen (umfassend Exons und Introns) exakt 551 bp vom ersten SNP in Exon 8 stromaufwärts in der Donor-Spleißstelle des Introns 6 zu Exon 7 des hsgkl-Gens lokalisiert ist und den Austausch eines T in ein C betrifft.

Es konnte weiterhin gezeigt werden, daß die in verschiedenen Körperpositionen gemessenen systolischen und diastolischen Blutdruckwerte alle in dem gleichen Ausmaß eine Abhängigkeit vom Genotyp des hsgkl-Gens zeigen (Tab. 4). Aus Tabelle 4 ist somit ersichtlich, daß die gefundenen Korrelationen zwischem dem gemessenen Blutdruck der Patienten und dem Auftreten der oben genannten Polymorphismen (SNPs) in ihren hsgk1-Genen tatsächlich statistisch relevant sind.

Weiterhin zeigen die beiden analysierten SNPs im hsgk1-Gen eine große Unausgeglichenheit in der Häufigkeit ihres korrelierten Auftretens (Tab. 5). Während die meisten CC-Träger des SNPs in Exon 8 auch TT-Träger des SNPs in Intron 6 sind (64%), ist dies umgekehrt nicht der Fall (nur 2% der Exon 8 TT-Träger sind auch Intron 6 CC-Träger).

Die erstmalig nachgewiesene Korrelation zwischen dem Blutdruck des Patienten und seiner individuellen genetischen Version des hsgk1-Genlocus zeigt, daß sich spezifische Antikörper oder Polynukleotide, die gegen hsgk1 gerichtet sind, zur Diagnose einer speziellen genetisch bedingten Neigung zur Hypertonie eignen. Diese spezielle genetisch verursachte Form der Hypertonie kann durch eine gesteigerte Expression der hsgk1, also durch Überexpression oder gegebenenfalls auch durch modifizierte funktionale Eigenschaften der hsgk1 charakterisiert sein.

Da die beiden homologen Kinasen der sgk-Familie, hsgk2 und hsgk3, ebenfalls den ENaC aktivieren, eignen sich erfindungsgemäß spezifische Antikörper und Polynukleotide, die gegen hsgk2 oder hsgk3 gerichtet sind, im gleichen Maß zur diagnostischen Analyse spezieller genetisch bedingter Formen der Hypertonie.

Der erfindungsgemäße Nachweis, daß das Auftreten der beiden SNPs im hsgk1-Gen mit einer Neigung zur Hypertonie korreliert, zeigt, daß insbesondere Polynukleotide, die die eine oder andere Version der beiden SNPs im hsgk1-Gen umfassen, sich in besonderem Maß zur Diagnose einer genetisch bedingten Form der Hypertonie durch Hybridisierung mit endogener DNA (cDNA oder genomische DNA) oder mRNA aus einer Körperprobe des Patienten eignen.

Ähnlich eignen sich nach den vorliegenden Ergebnissen auch Antikörper zur Diagnose einer genetisch bedingten Prädisposition der Hypertonie, die gegen spezifische Hypertonierelevante Polymorphismen (SNP) im hsgkl-Protein oder einem seiner humanen Homologen gerichtet sind. Solche SNPs, die auch auf Protein-Ebene zu einem Hypertonie-relevanten Polymorphismus führen, könnten insbesondere mit einer funktionalen Modifikation des hsgkl-Proteins einhergehen und so eine Prädisposition zur Hypertonie verursachen. Hierbei werden insbesondere die beiden mit der Neigung zur Hypertonie korrelierenden SNPs im hsgk1-Ge zur quantitativen Diagnose einer genetisch bedingten Hypertonie eingesetzt.

Bei diesem erfindungsgemäßen Diagnose-Verfahren werden als Körperproben des Patienten vorzugsweise Blutproben oder auch Speichelproben verwendet, die zelluläres Material umfassen und relativ wenig aufwendig vom Patienten gewonnen werden können. Andere Körperproben, die ebenfalls Zellen umfassen wie beispielsweise Gewebeproben u.ä., können jedoch auch verwendet werden. Aus diesem zellhaltigen Material der Körperproben kann dann entweder genomische DNA oder cDNA oder auch mRNA nach Standardmethoden (Sambrook-J and Russell-DW (2001) Cold Spring Harbor, NY, CSHL Press).

präpariert und gegebenenfalls amplifiziert werden und anschließend mit Polynukleotiden, die mit dieser genomischen DNA, cDNA oder auch mRNA spezifisch hybridisieren kann, unter stringenten Bedingungen hybridisiert. Weiterhin kann aus dem zellhaltigen Material der Körperproben (Blut, Speichel, Gewebe usw) auch ein Protein-Extrakt nach Standardmethoden (Sambrook-J and Russell-DW (2001) Cold Spring Harbor, NY, CSHL Press) isoliert werden, in dem dann das entsprechende sgk-Protein durch Inkubation mit einem Antikörper, der gegen dieses Protein gerichtet ist, quantitativ detektiert werden kann.

Bei dem Verfahren können vorzugsweise Antikörper gegen das hsgk1-Protein oder Polynukleotide, die mit genomischer DNA, c-DNA oder mRNA des hsgk1-Gens hybridisieren eingesetzt werden.

Bei dem erfindungsgemäßen Verfahren werden insbesondere Polynukleotide eingesetzt, welche mit DNA, c-DNA oder mRNA einer Version des SNP in Intron 6 des hsgk1-Gens oder einer Version des SNP in Exon 8 des hsgk1-Gens unter stringenten Bedingungen hybridisieren können.

Unter einer Hybridisierung unter stringenten Bedingungen wird in diesem Zusammenhang eine Hybridisierung unter solchen Hybridisierungsbedingungen bezüglich Hybridisierungstemperatur und Formamid-Gehalt der Hybridisierungslösung verstanden, wie sie in einschlägiger Fachliteratur. (Sambrook-J and Russell-DW (2001) Cold Spring Harbor, NY, CSHL Press) beschrieben wurde.

Zum Beispiel kann diagnostische Kit eine besonders bevorzugt Polynukleotide enthalten, die mit genormischer DNA, mit cDNA oder mit mRNA einer Version des SNP in Intron 6 (T→C) oder des SNP in Exon 8 (C→T) hybridisieren können.

Durch das nachfolgende Beispiele 2 wird die vorliegende Erfindung im Detail erläutert. Bespiel 1 ist vergleichenden Material.

## Beispiel 1

Es wurden 75 zweieigige Zwillingspärchen zur Korrelationsanalyse herangezogen (Busjahn et al., J Hypertens 1996, 14: 1195-1199; Busjahn et al., Hypertension 1997, 29: 165-170). Die Versuchspersonen waren alle Angehörige der deutsch-kaukasischen Rasse und stammten aus verschiedenen Teilen Deutschlands. Zur Verifikation der Zweieigigkeit und für weitere molekulargenetische Analysen wurde den Zwillingspärchen, sowie deren Eltern Blut entnommen. Jede teilnehmende Versuchsperson wurde zuvor ärztlich untersucht. Für keine der Versuchspersonen war eine chronisch-medizinische Erkrankung bekannt. Nach 5 min wurde der Blutdruck des Probanden in sitzender Position von einen ausgebildeten Arzt mit einem standardisierten Quecksilber-Sphygmomanometer gemessen (2 Messungen mit einem zeitlichen Intervall von 1 min). Der Mittelwert aus den beiden Messungen wurde als Blutdruckwert verwendet.

Der Vorteil von zweieigigen Zwillingen für Korrelationsstudien liegt darin, daß sie im Alter übereinstimmen und daß die äußeren Einflüsse auf ihre Phenotypen als minimal einzuschätzen sind (Martin et al., Nat Genet 1997,17: 387-392). Die Bedeutung von Zwillingsstudien bei der Aufklärung komplexer genetischer Krankheiten wurde kürzlich von Martin et al., 1997 beschrieben.

Die Zweieigigkeit der Zwillingspärchen wurde durch die Amplifikation von fünf Mikrosatelliten-Markem mit Hilfe der Polymerase Kettenreaktion (PCR) bestätigt. Bei dieser Analyse von Mikrosatelliten-Markern werden Desoxyribonucleinsäure (DNA) - Fragmente mit Hilfe spezifischer Oligonukleotide durch PCR amplifiziert, die bei verschiedenen menschlichen Individuen hochvariable Regionen beinhalten. Die hohe Variabilität in diesen Regionen des Genoms kann durch geringfügige Größenunterschiede der amplifizierten Fragmente detektiert werden, wodurch sich bei einer Diversität am entsprechenden Genort Doppelbanden, sogenannte Mikrosatelliten-Banden, nach gelelektrophoretischer Auftrennung der PCR-Produkte bilden (Becker et al., J Reproductive Med 1997, 42: 260-266).

Für die molekulargenetische Analyse des Zielgens, hier des hsgkl-Gens, wurden drei weitere Mikrosatelliten-Marker Regionen (d6s472, d6s1038, d6s270) in unmittelbarer Nähe des hsgkl-Locus durch PCR amplifiziert und anschließend mit den entsprechenden Proben des anderen Zwillings und der Eltern verglichen. Auf diese Weise konnte entschieden werden, ob die Zwillinge von ihren Eltern identische oder unterschiedliche Allele bezüglich des untersuchten Allels geerbt hatten. Die Korrelationsanalyse wurde mit Hilfe des sogenannten "structural equation modeling" (SEM) Modells durchgeführt (Eaves et al., Behav Genet 1996, 26: 519-525; Neale, 1997: Mx: Statistical modeling. Box 126 MCV, Richmond, VA 23298: Department of Psychiatry. 4th edition). Dieses Modell basiert auf Varianz-Kovarianz Matrizen der Test-Paare, die durch die Wahrscheinlichkeit, daß sie entweder keines, eines oder zwei identische Allele besitzen, charakterisiert sind. Die Varianz bezüglich des Phänotyps wurde aufgeteilt in eine Varianz, die auf dem genetischen Hintergrund aller Gene (A), eine Varianz, die auf dem genetischen Hintergrund des Zielgens (Q), hier des hsgkl-Gens, und der Varianz aufgrund äußerer Einflüsse (E) beruht.

$$VAR = A^2 + Q^2 + E^2$$

Für die drei möglichen Allelkombinationen $IBD_0$, $IBD_1$, $IBD_2$ (IBD = "identical by descent"; 0, 1 oder 2 identische Allele) wurde die Kovarianz eines Test-Paares wie folgt definiert:
$COV(IBD_0) = 0{,}5\ A^2$ $COV(IBD_1) = 0{,}5\ A^2 + 0{,}5\ Q^2$ $COV(IBD_2) = 0{,}5\ A^2 + Q^2$

Um die Korrelation zwischen der genetischen Ausstattung des hsgkl-Locus und dem Blutdruck des Probanden abzuschätzen, wurden die Differenzen zwischen Modellen, die die genetische Varianz bezüglich des Zielgens hsgk1 berücksichtigen bzw. nicht berücksichtigen, als $\chi^2$-Statistik berechnet. Für jedes Paar und jeden Genlocus wurden die Allelenverhältnisse durch das sogenannte "multipoint" Modell (MAPMAKER/SIBS; Kruglyak et al., Am J Hum Genet 1995, 57: 439-454) basierend auf den elterlichen Genotypen errechnet.

Die höhere Aussagekraft der Analysemethode, die auf einer Varianz-Kovarianz Abschätzung beruht, im Vergleich zur oben beschriebenen $\chi^2$-Statistik (S.A.G.E. Statistical Analysis for Genetic Epidemiology, Release 2.2. Computer program package, Department of Epidemiology and Biostatistics, Case Western Reserve University, Cleveland, OH, USA, 1996) wurde kürzlich in einer Simulationsstudie bestätigt (Fulker et al., Behav Gen 1996, 26: 527-532). Es wurde eine Irrtumswahrscheinlichkeit $p < 0{,}01$ akzeptiert, um eine signifikante Korrelation bezüglich der Kriterien von Lander und Kruglyak zu gewährleisten (Lander et al., Nat Genet 1995, 11: 241-246).

Die Ergebnisse dieser Korrelationsstudie zeigt Tabelle 1.

**Tabelle 1:**

| Phenotyp | max $\chi^2$ | p |
|---|---|---|
| systolischer Blutdruckwert (liegend) | 4,44 | 0,04 |
| diastolischer Blutdruckwert (liegend) | 14,36 | 0,0002 |
| systolischer Blutdruckwert (sitzend) | 5,55 | 0,019 |
| diastolischer Blutdruckwert (sitzend) | 4,92 | 0,027 |
| systolischer Blutdruckwert (stehend) | 1,91 | 0,17 |
| diastolischer Blutdruckwert (stehend) | 4,83 | 0,028 |

Wie aus der Tabelle 1 ersichtlich, beweisen die niedrigen Werte für die ermittelten Irrtumswahrscheinlichkeiten p, die die akzeptierte Irrtumswahrscheinlichkeit von $p < 0{,}01$ nicht oder nur geringfügig überschreiten, die direkte Korrelation zwischen der genetischen Varianz bezüglich des hsgk1-Genort und der phenotypisch ermittelten Varianz des gemessenen Blutdrucks.

<u>Beispiel 2</u>

Die genomische Organisation des hsgk 1-Gens wurde bereits beschrieben (Waldegger et al, Genomics,51,299 [1998]),
http://www.ensembl.org/Homo_sapiens/geneview?gene=ENSG0000018515).

Zur Identifizierung von SNPs, deren Auftreten für eine Prädisposition zur Ausbildung einer Hypertonie relevant sind, wurden zunächst die in Datenbanken publizierten SNPs im hsgk1-Gen danach untersucht, ob es sich um echte SNPs - und nicht um reine Sequenzierfehler - handelt und ob die SNPs ausreichend polymorph sind, um die Basis für einen diagnostischen Nachweis einer Prädisposition zur Hypertonie zu stellen. Der SNP rs 1057293 in Exon 8, der einen Austausch eines C in ein T betrifft, erfüllte die geforderten Voraussetzungen (http://www.ensembl.org/Homo_sapiens/snpview?snp=1057293; http://www.ncbi.nlm.nih.gov/SNP/snp_refcgi?type=rs&rs=1057293). Weiterhin wurde ein zweiter SNP durch direkte Sequenzierung identifiziert, der im hsgk1-Gen exakt 551 bp vom ersten SNP entfernt in der Donor-Spleißstelle des Introns 6 zu Exon 7 lokalisiert ist und den Austausch eines T in ein C betrifft. Diese beiden SNPs in Intron 6 (T→C) und in Exon 8 (C→T) wurden wie im folgenden beschrieben analysiert.

Nach der PCR-Amplifikation wurde jeweils 1 Unit Alkaline Phosphatase und 1 Unit Exonuklease I hinzugefiigt, um die PCR-Primer zu degenerieren und die dNTPs zu dephosphorylieren. Die PCR wurde unter den folgenden Bedingungen durchgeführt: 95° C für 10 min, dann 35 Zyklen bei 95° für 15 sec, gefolgt von 62°C for 15 sec, gefolgt von 72°C für 30 sec, und einem Extensionsschritt bei 72° C für 10 min in einem 9600 Thermocycler (Applied Biosystems).

Die Mini-Sequenzierreaktionen wurden mit den Primern für den Intron 6 SNP (T→C) 5'-CTC CTT GCA GAG TCC GAA und für den Exon 8 SNP (C→T) 5'-ACC AAG TCA TTC TGG GTT GC durchgeführt. 0,15 pmol aufgereinigtes PCR-Produkt wurde bei der Sequenzierungs-PCR als Template eingesetzt. Für die Sequenzierungs-PCR wurden 25 Amplifikationszyklen durchgeführt mit den folgenden Einzelschritten: Denaturierung 10 s bei 96 °C, Annealingschritt 10 s bei 50 °C und Extensionsschritt 30 s bei 60 °C in einem 9600 Thermocycler.

Bei denselben Patienten, deren SNP-Genotyp des hsgkl-Gens bestimmt wurde, wurden die systolischen und diastolischen Blutdruckwerte in liegender, stehender und sitzender Position gemessen, um eine eventuelle Korrelation zwischen SNP-Genotyp des hsgk1-Gens und dem Blutdruck zu ermitteln.

Tabelle 2 zeigt einige demographische Zwillingsdaten und die Resultate der Korrelationsanalyse zwischen der genetischen Ausstattung des hsgk1-Genlocus und dem gemessenen Blutdruck. Ein starker genetischer Einfluß auf den gemessenen Blutdruck in allen Positionen konnte bei den Versuchspersonen nachgewiesen werden.

**Tabelle 2:**

| Phänotyp | eineiige Zwillinge | zweieiige Zwillinge | $a^2$ ($r_{eineiig}/r_{zweieig}$) | p (Korrelation) |
|---|---|---|---|---|
| N | 200 | 132 | | |
| Alter y | 29±12 | 31±12 | | |
| Geschlecht (M/F) | 52/148 | 85/47 | | |
| Größe (cm) | 169±8 | 170±8 | | |
| Gewicht (kg) | 65±11 1 | 67±12 | | |
| body mass index (BMI) Gewicht/Größe$^2$ (kg/m$^2$) | 22.4±3.5 | 22.8±3.4 | | |
| Systolischer Blutdruck (liegend) (mm Hg) | 128±17 | 124±14 | 0.69(0.69/0.31) | 0.04 |
| Diastolischer Blutdruck (liegend) (mm Hg) | 71±12 | 71±11 | 0.66 (0.66/0.42) | 0.0002 |
| Systolischer Blutdruck (sitzend) (mm Hg) | 125±16 | 123±13 | 0.74 (0.74/0.38) | 0.019 |
| Diastolischer Blutdruck (sitzend) (mm Hg) | 73±11 | 73±10 | 0.72(0.72/0.51) | 0.027 |
| Systolischer Blutdruck (stehend) (mm Hg) | 124±15 | 122±14 | 0.67 (0.66/0.48) | 0.04 |
| Diastolischer Blutdruck (stehend) (mm Hg) | 80±10 | 79±10 | 0.64 (0.63/0.40) | 0.0002 |

Tabelle 3 zeigt weitere Resultate der erfindungsgemäßen Korrelationsstudien. Die ermittelten Allelhäufigkeiten für den SNP in Exon 8 liegen bei C 91% und T 9% und für den SNP in Intron 6 bei T 79% und C 21 % (das Hardy-Weinberg Gleichgewicht wurde für beide Polymorphismen erhalten).

Die gemessenen Blutdruckwerte zeigten in allen Positionen (sitzend, liegend, stehend) die gleichen Trends. Homozygote CC-Träger und heterozygote CT-Träger des SNPs in Exon 8 zeigten keine voneinander abweichende Blutdruckwerte, zeigten jedoch deutlich niedrigere systolische und diastolische Blutdruckwerte als homozygote TT-Träger des SNPs in Exon 8.

Die entsprechenden Resultate der Korrelationsstudien sind für den SNP in Intron 6 im Vergleich zum SNP in Exon 8 weniger konsistent. Es zeigte sich jedoch, daß homozygote CC-Träger des SNPs in Intron 6 generell niedrigere Blutdruckwerte aufweisen als homozygote TT-Träger und als heterozygote TC-Träger des SNPs in Intron 6.

**Tabelle 3:**

| Phänotyp | 1. SNP in Exon 8 CC | 1. SNP in Exon 8 CT | 1. SNP in Exon 8 TT | 1. SNP in Exon 8 CC/CT | 2. SNP in Intron 6 TT | 2. SNP in Intron 6 CT | 2. SNP in Intron 6 CC | 2. SNP in Intron 6 TT/CT |
|---|---|---|---|---|---|---|---|---|
| systol. Blutdruck (liegend) | 125 ± 15 | 125 ± 18 | 132 ± 14 | 125 ± 16 | 125 ± 16 | 128 ± 18 | 119 ± 6 | 126±16 |
| diastol. Blutdruck (liegend) | 70 ± 10 | 72 ± 13 | 74 ± 12 | 71 ± 11 | 71 ± 10 | 72 ± 13 | 67 ± 10 | 71 ± 11 |
| systol. Blutdruck (sitzend) | 124 ± 14 | 123 ± 15 | 129 ±13 | 124 ± 14 | 124 ± 14 | 125 ± 17 | 117 ± 6 | 124 ± 14 |
| diastol. Blutdruck (sitzend) | 72 ± 10 | 74 ± 10 | 79 ± 9 | 73 ± 10 | 73 ± 10 | 74 ± 11 | 72 ± 9 | 73 ± 10 |
| systol. Blutdruck (stehend) | 123±15 | 123 ± 14 | 129 ± 13 | 123 ± 15 | 123 ± 14 | 126 ± 16 | 119 ± 8 | 123 ± 15 |
| diastol. Blutdruck (stehend) | 79 ± 10 | 81 ± 10 | 84 ± 8 | 80 ± 10 | 80 ± 10 | 82 ± 11 | 78 ± 8 | 80 ± 10 |

Tabelle 4 zeigt im Detail, daß sowohl für den systolischen als auch für den diastolischen Blutdruckwert die genetische Ausstattung des SNPs in Intron 6 weitgehend gleichermaßen signifikant ist, und zwar unabhängig von der Position, in der der Blutdruck gemessen wurde (sitzend, stehend, liegend). Die Resultate für die Signifikanz der genetischen Ausstattung des SNPs in Exon 8 sind ähnlich, die Assoziation der Signifikanz zwischen den gemessenen systolischen und diastolischen Blutdruckwerten in den verschiedenen Positionen ist jedoch etwas weniger ausgeprägt als bei dem SNP in Intron 6.

**Tabelle 4:**

| Phänotyp | 2. SNP in Intron 6 | 1. SNP in Exon 8 |
|---|---|---|
| Systolischer Blutdruck (liegend) | <0.01 | <0.05 |
| Diastolischer Blutdruck (liegend) | <0.05 | 0.08 |
| Systolischer Blutdruck (sitzend) | <0.05 | <0.05 |
| Diastolischer Blutdruck (sitzend) | <0.01 | 0.08 |
| Systolischer Blutdruck (stehend) | <0.05 | 0.07 |
| Diastolischer Blutdruck (stehend) | <0.05 | 0.09 |

Wie aus Tabelle 5 ersichtlich ist, besteht ein starkes Korrelationsungleichgewicht zwischen den beiden analysierten SNPs: während die meisten CC-Träger des SNPs in Exon 8 auch TT-Träger des SNPs in Intron 6 sind (64%), ist dies umgekehrt nicht der Fall (nur 2% der Exon 8 TT-Träger sind auch Intron 6 CC-Träger).

**Tabelle 5:**

| | Intron 6 TT | Intron 6 TC | Intron 6 CC |
|---|---|---|---|
| Exon 8 CC | 197 (64%) | 59 (19%) | 3 (1%) |
| Exon 8 CT | 2 (1%) | 30 (10%) | 11 (4%) |
| Exon 8TT | 0 (0%) | 0(0%) | 6 (2%) |

**Patentansprüche**

1. In vitro Verfahren zur Diagnose einer genetisch bedingten Prädisposition für Hypertonie, **gekennzeichnet dadurch, dass** in einer Körperprobe eines Patienten

   (a) der Hypertonie-korrelierte SNP in Exon 8 (C → T) im hsgk1 Gen, wie in SEQ ID No: 1 gezeigt;
   (b) der Hypertonie-korrelierte SNP im Intron 6 (T → C) im hsgk1 Gen, der 551bp stromaufwärts von Exon 8 SNP aus (a) lokalisiert ist; oder
   (c) der Hypertonie-korrelierte SNP aus (a) und der Hypertonie-korrelierte SNP aus (b)

   bestimmt werden.

2. Verwendung des Hypertonie-korrelierten SNP in Exon 8 (C → T) im hsgk1 Gen, wie in SEQ ID No: 1 gezeigt, des Hypertonie-korrelierten SNP im Intron 6 (T → C) im hsgk1 Gen, der 551bp stromaufwärts von Exon 8 SNP aus (a) lokalisiert ist, oder beider SNPs zur Herstellung eines Diagnostikums zur Diagnose einer genetisch bedingten Prädisposition für Hypertonie.

**Claims**

1. In vitro method for diagnosing a genetically determined predisposition to hypertension, **characterized in that**, in a body sample from a patient,

   a) the hypertension-correlated SNP in the exon 8 (C→T) of the hsgk1 gene, as shown in SEQ ID No:1;
   b) the hypertension-correlated SNP in the intron 6 (T→C) of the hsgk1 gene which is located 551bp upstream from the exon 8 SNP of (a); or
   c) the hypertension-correlated SNP of (a) and the hypertension-correlated SNP of (b) are determined.

2. Use of the hypertension-correlated SNP in the exon 8 (C→T) of the hsgk1 gene, as shown in SEQ ID No:1, the hypertension-correlated SNP in the intron 6 (T→C) of the hsgk1 gene which is located 551bp upstream from the exon 8 SNP of (a), or of both SNPs for producing a diagnostic composition for diagnosing a genetically determined predisposition to hypertension.

**Revendications**

1. Procédé in vitro pour le diagnostic d'une prédisposition d'origine génétique à l'hypertonie, **caractérisé en ce que**, dans un échantillon corporel d'un patient, on détermine

   (a) le SNP (mutation ponctuelle isolée) corrélé à l'hypertonie dans l'intron 8 (C → T) du gène hsgk1, tel que présenté dans SEQ ID N°: 1 ;
   (b) le SNP corrélé à l'hypertonie dans l'intron 6 (T → C) du gène hsgk1 localisé 551bp en amont du SNP de l'Exon 8 de (a), ou
   (c) le SNP corrélé à l'hypertonie de (a) et le SNP corrélé à l'hypertonie de (b).

**2.** Utilisation du SNP corrélé à l'hypertonie de l'Exon 8 (C → T) du gène hsgk1, tel que présenté dans SEQ ID N°: 1, du SNP corrélé à l'hypertonie de l'Intron 6 (T → C) du gène hsgk1, qui est localisé 551bp en amont du SNP de l'Exon 8 de (a), ou des deux SNP, pour préparer un produit diagnostique destiné au diagnostic d'une prédisposition d'origine génétique à l'hypertonie.

# Figure1:

1 ENSE00000798789 AL135839.15.1.113673 -1 27517 27634 118 bp
GGTCTTTGAGCGCTAACGTCTTTCTGTCTCCCCGCGGTGGTGATGACGGT
GAAAACTGAGGCTGCTAAGGGCACCCTCACTTACTCCAGGATGAGGGGCA
TGGTGGCAATTCTCATCG
2 ENSE00000798790 AL135839.15.1.113673 -1 27296 27371 76 bp
CTTTCATGAAGCAGAGGAGGATGGGTCTGAACGACTTTATTCAGAAGATT
GCCAATAACTCCTATGCATGCAAACA
3 ENSE00000798791 AL135839.15.1.113673 -1 26790 26865 76 bp
CCCTGAAGTTCAGTCCATCTTGAAGATCTCCCAACCTCAGGAGCCTGAGC
TTATGAATGCCAACCCTTCTCCTCCA
4 ENSE00000798792 AL135839.15.1.113673 -1 26247 26351 105 bp
CCAAGTCCTTCTCAGCAAATCAACCTTGGCCCGTCGTCCAATCCTCATGC
TAAACCATCTGACTTTCACTTCTTGAAAGTGATCGGAAAGGGCAGTTTTG
GAAAG
5 ENSE00000798793 AL135839.15.1.113673 -1 26059 26142 84 bp
GTTCTTCTAGCAAGACACAAGGCAGAAGAAGTGTTCTATGCAGTCAAAGT
TTTACAGAAGAAAGCAATCCTGAAAAAGAAAGAG
6 ENSE00000798794 AL135839.15.1.113673 -1 25808 25939 132 bp
GAGAAGCATATTATGTCGGAGCGGAATGTTCTGTTGAAGAATGTGAAGCA
CCCTTTCCTGGTGGGCCTTCACTTCTCTTTCCAGACTGCTGACAAATTGT
ACTTTGTCCTAGACTACATTAATGGTGGAGAG
7 ENSE00000798795 AL135839.15.1.113673 -1 25447 25559 113 bp
TTGTTCTACCATCTCCAGAGGGAACGCTGCTTCCTGGAACCACGGGCTCG
TTTCTATGCTGCTGAAATAGCCAGTGCCTTGGGCTACCTGCATTCACTGA
ACATCGTTTATAG
8 ENSE00000798796 AL135839.15.1.113673 -1 24978 25101 124 bp
AGACTTAAAACCAGAGAATATTTTGCTAGATTCACAGGGACACATTGTCCTT
ACTGA[redacted]TTCGGACTCTGGAAGGAGAACATTGAACACAACAGCACAACA
TCCACCTTCTGTGGCACGCCGGAG
9 ENSE00000798797 AL135839.15.1.113673 -1 24422 24517 96 bp
TATCTCGCACCTGAGGTGCTTCATAAGCAGCCTTATGACAGGACTGTGGA
CTGGTGGTGCCTGGGAGCTGTCTTGTATGAGATGCTGTATGGCCTG
10 ENSE00000798798 AL135839.15.1.113673 -1 23808 23963 156 bp
CCGCCTTTTTATAGCCGAAACACAGCTGAAATGTACGACAACATTCTGAA
CAAGCCTCTCCAGCTGAAACCAAATATTACAAATTCCGCAAGACACCTCC
TGGAGGGCCTCCTGCAGAAGGACAGGACAAAGCGGCTCGGGGCCAAGGAT
GACTTC
11 ENSE00000798799 AL135839.15.1.113673 -1 23611 23700 90 bp
ATGGAGATTAAGAGTCATGTCTTCTTCTCCTTAATTAACTGGGATGATCT
CATTAATAAGAAGATTACTCCCCCTTTTAACCCAAATGTG
12 ENSE00000798800 AL135839.15.1.113673 -1 22037 23220 1184 bp
AGTGGGCCCAACGACCTACGGCACTTTGACCCCGAGTTTACCGAAGAGCC
TGTCCCCAACTCCATTGGCAAGTCCCCTGACAGCGTCCTCGTCACAGCCA
GCGTCAAGGAAGCTGCCGAGGCTTTCCTAGGCTTTTCCTATGCGCCTCCC
ACGGACTCTTTCCTCTGAACCCTGTTAGGGCTTGGTTTTAAAGGATTTTA
TGTGTGTTTCCGAATGTTTTAGTTAGCCTTTTGGTGGAGCCGCCAGCTGA
CAGGACATCTTACAAGAGAATTTGCACATCTCTGGAAGCTTAGCAATCTT
ATTGCACACTGTTCGCTGGAAGCTTTTTGAAGAGCACATTCTCCTCAGTG
AGCTCATGAGGTTTTCATTTTTATTCTTCCTTCCAACGTGGTGCTATCTC
TGAAACGAGCGTTAGAGTGCCGCCTTAGACGGAGGCAGGAGTTTCGTTAG
AAAGCGGACGCTGTTCTAAAAAAGGTCTCCTGCAGATCTGTCTGGGCTGT
GATGACGAATATTATGAAATGTGCCTTTTCTGAAGAGATTGTGTTAGCTC
CAAAGCTTTTCCTATCGCAGTGTTTCAGTTCTTTATTTTCCCTTGTGGAT
ATGCTGTGTGAACCGTCGTGTGAGTGTGGTATGCCTGATCACAGATGGAT
TTTGTTATAAGCATCAATGTGACACTTGCAGGACACTACAACGTGGGACA
TTGTTTGTTTCTTCCATATTTGGAAGATAAATTTATGTGTAGACTTTTTT
GTAAGATACGGTTAATAACTAAAATTTATTGAAATGGTCTTGCAATGACT
CGTATTCAGATGCTTAAAGAAAGCATTGCTGCTACAAATATTTCTATTTT
TAGAAAGGGTTTTTATGGACCAATGCCCCAGTTGTCAGTCAGAGCCGTTG
GTGTTTTTCATTGTTTAAAATGTCACCTGTAAAATGGGCATTATTTATGT
TTTTTTTTTGCATTCCTGATAATTGTATGTATTGTATAAAGAACGTCTG
TACATTGGGTTATAACACTAGTATATTTAAACTTACAGGCTTATTTGTAA
TGTAAACCACCATTTTAATGTACTGTAATTAACATGGTTATAATACGTAC
AATCCTTCCCTCATCCCATCACACAACTTTTTTTGTGTGTGATAAACTGA
TTTTGGTTTGCAATAAAACCTTGAAAAATATTTA